# EUROPÄISCHE PATENTSCHRIFT

(11) **EP 1 094 998 B1**
(45) Veröffentlichungstag und Bekanntmachung des Hinweises auf die Patenterteilung: **11.12.2002**
(21) Anmeldenummer: 99932740.6
(22) Anmeldetag: 26.06.1999
(51) Int. Cl.: C07C 9/22

(54) **VERFAHREN ZUR HERSTELLUNG VON PHYTOSQUALAN**
METHOD FOR PRODUCING PHYTOSQUALANE
PROCEDE DE PREPARATION DE PHYTOSQUALANE

(30) Priorität: 06.07.1998 DE 19830171
(43) Veröffentlichungstag der Anmeldung: 02.05.2001
(73) Patentinhaber: Cognis Deutschland GmbH & Co. KG, 40589 Düsseldorf (DE)
(72) Erfinder: PI SUBIRANA, Rafael, E-08400 Granollers (ES); PONSATI OBIOLS, Oriol, E-08005 Barcelona (ES); BIGORRA LLOSAS, Joaquin, E-08203 Sabadell (ES)
(86) Internationale Anmeldenummer: EP9904455
(87) Internationale Veröffentlichungsnummer: WO00001645

(56) Entgegenhaltungen:
- DE-A- 4 316 620
- GB-A- 2 217 729

## Beschreibung

### Gebiet der Erfindung

Die Erfindung befindet sich auf dem Gebiet der kosmetischen Ölkörper und betrifft ein verbessertes Verfahren zur Herstellung von pflanzlichem Squalan durch Derivatisierung von Rückständen aus der Pflanzenölherstellung.

### Stand der Technik

Squalan (2,6,10,15,19,23-Hexamethyl-2,6,10,14,18,22-tetracosan) gehört zur Gruppe der acyclischen Triterpene, wird gewöhnlich durch Hydrierung von Squalen (2,6,10,15,19,23-Hexamethyl-2,6,10,14,18,22-tetracoshexaen) gewonnen und dient als besonders unpolarer Ölkörper für kosmetische und pharmazeutische Anwendungen. Der ungesättigte Grundkörper, also das Squalen, ist zwar auf synthetischem Wege, beispielsweise durch Umsetzung von Hexaphenyl-1,4-butandiyldiphosphoniumdibromid mit 6,10-Dimethyl-5,9-undecadien-2-on erhältlich, die Umsetzung ist jedoch technisch sehr aufwendig. Auf diesem Wege hergestelltes Squalen bzw. Squalan ist daher sehr teuer, zumal der geforderte Reinheitsgrad für eine kommerzielle Verwertung kaum zu erreichen ist.

Aus diesem Grunde bedient man sich zur Herstellung von Squalen bzw. Squalan üblicherweise natürlicher Quellen, wie beispielsweise Haifischleberöl oder Preßrückständen bei der Gewinnung von Pflanzenölen, insbesondere von Olivenöl. Seetierisches Squalan, welches man beispielsweise durch Extraktion erhalten kann, ist für kosmetische oder pharmazeutische Anwendung jedoch weniger geeignet, da es in der Regel trotz aller Desodorierungsmaßnahmen einen störenden Geruch aufweist und unerwünschte Verunreinigungen enthält. Zu den Unterschieden zwischen tierischem und pflanzlichem Squalan (Phytosqualan) sei auf die Übersicht von A.Gasparoli in **Riv.Italiana.Sostanze Grasse, Vol. LXXIII, S.293 (1996)** verwiesen.

Auch die Herstellung von pflanzlichem Squalan gehört grundsätzlich zum Stand der Technik. Aus der spanischen Patentanmeldung **ES 2002428** (Hispano Quimica) ist ein Verfahren zur Herstellung von pflanzlichem Squalan bekannt, bei dem man einen Preßrückstand der Olivenölherstellung zunächst partiell hydriert und dabei eine Mischung aus Squalen, Squalan, Kohlenwasserstoffen und ungesättigten Fettsäuren gewinnt. Dieses Gemisch wird im zweiten Schritt durch fraktionierte Kristallisation in ein flüssiges Konzentrat überführt, dessen Anteil an freien Fettsäuren durch Zugabe von Base verseift und abgetrennt wird. Anschließend wird die zurückbleibende Fraktion hydriert, wobei Squalen in Squalan umgewandelt wird. Abschließend werden die in der Fraktion noch enthaltenen Paraffine destillativ abgetrennt. Gegenstand der spanischen Patentanmeldung **ES 2011259** (Hispano Quimica) ist ein ähnliches Verfahren zur Herstellung von Phytosqualan, bei der man den sauren Preßrückstand durch Destillation und Verseifung von Fettsäuren befreit, den Rückstand hydriert, die Paraffine ausfriert ("Winterisierung") und das reine Phytosqualan durch abschließende Destillation erhält. Schließlich wird in der spanischen Patentschrift **ES 2063697** (Hispano Quimica) vorgeschlagen, die Deparaffination durch Wäsche mit Schwefelsäure durchzuführen. Aus der japanischen Patentanmeldung **JP-A Hei 09/176057** (Koyo Fine Chemicals) ist ein Verfahren zur Herstellung von Squalan bekannt, bei dem man eine bei der destillativen Reinigung von Olivenöl anfallende Fraktion mit einem Gehalt von 35 Gew.-% Squalen und 50 Gew.-% freien Fettsäuren von den Säuren befreit und hydriert. Anschließend wird die squalanhaltige Fraktion in Isopropylalkohol gelöst und durch Zugabe von Harnstoff deparaffiniert. Nach Abtrennung des Lösemittels wird das Squalan in einer Reinheit von 93 % erhalten. Ein weiteres Verfahren zur Herstellung von pflanzlichen Squalanen ist aus der japanischen Patentanmeldung **JP-A Hei 06/306387** (Nisshin Oil Mills) bekannt. Dabei werden Rückstände von squalenhaltigen Ölen, wie beispielsweise Olivenöl, Sojaöl oder Palmöl zunächst hydriert und dann durch fraktionierte Kristallisation aufgereinigt. Weiterhin wird in der japanischen Patentanmeldung **JP-A Hei 06/306388** (Nisshin Oil Mills) vorgeschlagen, die sauren Destillate nach der Hydrierung in organischen Lösemitteln aufzunehmen und unter Druck durch Molekularsiebe zu filtern. Schließlich ist aus der deutschen Patentanmeldung **DE-A1 4316620** (Müller Extrakt) ein mehrstufiges Verfahren zur Herstellung von pflanzlichem Squalen bekannt, bei dem man Rückstände aus der Olivenölherstellung verseift, spaltet, die Fettsäuren verestert und den Wertstoff durch Extraktion mit über- oder unterkritischen Gasen erhält.

Die genannten Verfahren weisen jedoch eine Reihe von Nachteilen auf: die Reinheit der Squalane läßt insbesondere im Hinblick auf den Gehalt an Paraffinen zu wünschen übrig (Schwefelsäurewäsche), die Durchführung ist technisch aufwendig (Molekularsiebe, Gasextraktion), ferner werden Koppelprodukte (Fettsäureester) hergestellt, für die es wegen der darin enthaltenen Verunreinigungen praktisch keine Verwendung gibt.

Die komplexe Aufgabe der Erfindung hat folglich darin bestanden, ein Verfahren zur Herstellung von Phytosqualan zur Verfügung zu stellen, welches die Nachteile des Stands der Technik zuverlässig vermeidet und auf möglichst einfachem Wege Phytosqualan einer pharmazeutischen Qualität liefert, wobei die Bildung von unnützen Koppelprodukten umgangen wird.

### Beschreibung der Erfindung

Gegenstand der Erfindung ist ein Verfahren zur Herstellung von Phytosqualan, welches sich dadurch auszeichnet, daß man
(a) einen phytosqualenhaltigen Rückstand der Pflanzenölherstellung mit einem Polyol zur Derivatisierung des Fettsäureanteils verestert,
(b) die resultierende Reaktionsmischung in eine Polyolesterfraktion (Rückstand) und ein phytosqualenhaltiges Destillat trennt,
(c) das Destillat unter Erzeugung einer squalanreichen Fraktion hydriert,
(d) das resultierende flüssige Hydrierprodukt einer fraktionierten Kristallisation unterwirft,
(e) die von Paraffinen befreite flüssige Phase durch Behandlung mit Schwefeltrioxid, Chlorsulfonsäure oder Amidosulfonsäure behandelt, und
(f) aus dem Rückstand das reine Phytosqualan durch Wäsche und Desodorierung gewinnt.

Gegenüber dem Stand der Technik besteht der wesentlichen Unterschied des erfindungsgemäßen Verfahrens darin, daß man die saure Wäsche nicht mit Schwefelsäure, sondem Schwefeltrioxid, Chlorsulfonsäure oder Amidosulfonsäure durchführt. Ein weiterer Unterschied besteht darin, daß man die Winterisierung nicht auf der Stufe des Squalens (also vor der Hydrierung), sondem auf der Stufe des Squalans durchführt. Durch diese Maßnahmen wird der Gehalt an unerwünschten Kohlenwasserstoffen gegenüber dem Stand der Technik auf unter 0,5 Gew.-% begrenzt. Ein weiterer Vorteil besteht darin, daß die Polyole mit den Fettsäuren Ester bilden, welche beispielsweise als Textilhilfsmittel verwendet werden können und somit nicht entsorgt werden müssen.

### Ausgangsstoffe

Als Ausgangsstoffe für die Herstellung des pflanzlichen Squalans dienen vorzugsweise Rückstände, die bei der Herstellung von Olivenöl anfallen und ansonsten verbrannt werden. Altemativ können entsprechende Rückstände aus der Herstellung von anderen squalenhaltigen Pflanzenölen, wie z.B. Sojaöl, Reisschalenöl oder Palmöl eingesetzt werden. Das erfindungsgemäße Verfahren nutzt daher ansonsten wertlose Abfallstoffe zur Gewinnung wohlfeiler Rohstoffe für Kosmetik und Pharmazie. Bei den Rückständen, die unter der Bezeichnung "skam" bekannt sind, handelt es sich um komplexe Mischungen, welche überwiegend, d.h. zu 60 bis 90 Gew.-% aus Fettstoffen, vor allem gesättigten und vorzugsweise ungesättigten Fettsäuren, Tri-, Di- und Monoglyceriden bestehen und daneben noch einen hohen Anteil, d.h. 10 bis 40, in der Regel um die 20 Gew.-% unverseifbare Anteile, d.h. Kohlenwasserstoffe aufweisen. Bei diesen handelt es sich beispielsweise um Paraffine, Olefine, Terpene, Tocopherole, Sterole und dergleichen, wobei der Gehalt an Squalen in der Regel bei etwa 5 Gew.-% - bezogen auf die Einsatzstoffe - liegt. Derartige Rückstandsfraktionen sind auf dem Markt frei erhältlich.

### Veresterung bzw. Umesterung

Der erste Schritt auf dem Wege zur Herstellung von Phytosqualan besteht darin, die Trennung zwischen den Fettstoffen und den Kohlenwasserstoffen zu erleichtern. Dazu werden die freien Fettsäuren und Glyceride mit Polyolen verestert bzw. umgeestert. Als Polyolkomponenten kommen dabei in Frage:
- technische Oligoglyceringemische wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest, wie beispielsweise Methyl- und Butylglucosid;
- Zuckeralkohole wie beispielsweise Sorbit oder Mannit,
- Zucker, wie beispielsweise Glucose oder Saccharose;
- Aminozucker, wie beispielsweise Glucamin.

Vorzugsweise werden als Polyole Trimethylolpropan oder Pentaerythrit eingesetzt, da die resultierenden Ester anschließend als Textilhilfsmittel oder Schmierstoffe eingesetzt werden können. Die Veresterung/Umesterung kann in an sich bekannter Weise durchgeführt werden, d.h. bezogen auf die zur Verfügung stehenden Acylreste setzt man das Polyol - hier bezogen auf Hydroxylgruppen - im Überschuß ein, beispielsweise im molaren Verhältnis 1 : 2 bis 1 : 3. Gegenüber dem Einsatz von Monoalkoholen hat dies den Vorteil, daß bei ausreichend langen Reaktionszeiten mindestens eine Hydroxylgruppe des Polyols verestert und das Polyol vollständig in eine schwerflüchtige Verbindung überführt wird. Als Katalysatoren für die Veresterung/Umesterung kommen die aus dem Stand der Technik bekannten Verbindungen in Frage, wobei der Einsatz von Zinkseifen besonders bevorzugt ist. Die Einsatzmenge der Katalysatoren kann 0,1 bis 5 und vorzugsweise 1 bis 2 Gew.-% betragen. Es empfiehlt sich, die Umsetzung bei Temperaturen im Bereich von 130 bis 220 und vorzugsweise 150 bis 200°C sowie verminderten Drücken im Bereich von 0,9 bis 0,05, vorzugsweise 0,5 bis 0,1 bar durchzuführen und dabei während der Reaktionszeit (typischerweise 5 bis 8 h), die Temperatur zu steigem und den Druck abzusenken. Es versteht sich, daß zur Verschiebung des Gleichgewichtes auf die Seite der Polyole das Kondensationswasser kontinuierlich abdestilliert wird. Falls gewünscht, kann die Umsetzung unter Stickstoffabdeckung erfolgen.

### Abtrennung der Leichtsieder vom Polyolester

Es wurde schon darauf hingewiesen, daß die Veresterung mit Monoalkoholen wie beispielsweise Methanol zwar grundsätzlich möglich ist, jedoch zu Methylestern führt, die wegen ihres Gehaltes an Kohlenwasserstoffen kaum Verwendung finden. Für Polyolester, die man beispielsweise als Schmierstoffe verwendet, ist der Kohlenwasserstoffgehalt unerheblich, in manchen Fällen sogar nützlich. Ein weiterer Vorteil für den Einsatz von Polyolen besteht darin, daß die resultierenden Ester einen sehr viel höheren Siedepunkt als beispielsweise Methylester aufweisen, so daß die Abtrennung der Kohlenwasserstoffe (Siedepunkt Squalen : 184°C/25 mbar) wesentlich erleichtert wird. Die Destillation kann als solche wieder in an sich bekannter Weise durchgeführt werden, wobei typische Bedingungen 250 bis 290°C/5 bis 25 mbar sind. Es empfiehlt sich, die Abtrennung der Kohlenwasserstoffe über eine gepackte Kolonne oder in einem Fallfilm- bzw. Dünnschichtverdampfer vorzunehmen und gegebenenfalls ein Rücklaufverhältnis von 1 : 2 bis 1 : 10 einzustellen. Während der Polyolester also als Rückstand verbleibt, wird das Destillat weiterverarbeitet.

### Hydrierung

Mit der Hydrierung wird das Ziel verfolgt, ungesättigte Anteile in der Kohlenwasserstoffraktion zu härten und dabei insbesondere Squalen in Squalan zu überführen. Die Härtung kann wiederum in an sich bekannter Weise erfolgen, also beispielsweise in Gegenwart von Nickel-, Palladium- oder Platinkatalysatoren. Gute Ergebnisse werden z.B. mit Palladium/Aktivkohle (1 bis 5 Gew.-%)-Katalysatoren erzielt. Die Einsatzmenge der Katalysatoren kann im Bereich von 0,01 bis 1 Gew.-% - bezogen auf den Einsatzstoff - liegen und beträgt in der Regel etwa 0,1 bis 0,5 Gew.-%. Die Hydrierung wird üblicherweise bei 150 bis 200 und vorzugsweise 180 bis 190°C und 1 bis 25; vorzugsweise 15 bis 20 bar durchgeführt. Nach Reaktionszeiten von in der Regel 1 bis 5 h wird der Autoklav abgekühlt, entspannt und der Katalysator abfiltriert. Die lodzahlen der Hydrierprodukte liegen dabei typischerweise unter 0,5.

### Fraktionierte Kristallisation ("Winterisierung")

Ziel der sich anschließenden fraktionierten Kristallisation, die auch nach einem ihrer Erfinder als "Winterisierung" bezeichnet wird, ist es, Spuren von Paraffinen, die die bisherigen Reinigungsschritte überstanden haben, abzutrennen. Hierzu werden die Paraffine ausgefroren, indem man die flüssigen Hydrierprodukte auf Temperaturen im Bereich von -15 bis -25°C, vorzugsweise -15 bis -20°C abkühlt und die nach mehrstündiger Lagerung ausgefallenen Paraffine über ein Filter abtrennt. Zur Erleichterung der Kristallisation empfiehlt es sich, die Hydrierprodukte zuvor in einem inerten Lösemittel, beispielsweise n-Hexan, aufzunehmen. Allerdings muß das Lösemittel nach der Kristallisation wieder abgetrennt werden. Die Deparaffinierung kann gegebenenfalls auch im Anschluß an die Sulfierung erfolgen.

### Sulfierung

Nach Abtrennung der Paraffine sind in der nun schon stark aufgereinigten Squalanfraktion als hauptsächliche Verunreinigungen nur noch einige Fettstoffe enthalten. Diese werden entfernt, indem man sie mit geeigneten Sulfiermitteln behandelt und dabei in wasserlösliche Verbindungen überführt. Als Sulfiermittel kommen dabei gasförmiges Schwefeltrioxid (in der Regel als 0,1 bis 5 Vol.-%ige Verdünnung in Stickstoff oder Luft), Amidosulfonsäure und insbesondere Chlorsulfonsäure in Frage. Die Zusatzmenge kann 0,1 bis 10, vorzugsweise aber 0,2 bis 1 Gew.-% - bezogen auf die Squalanfraktion - betragen. In der Praxis empfiehlt es sich, die Kohlenwasserstoffmischung vorzulegen und die Chlorsulfonsäure oder die Amidosulfonsäure einzurühren bzw. das gasförmige Schwefeltrioxid über ein Gaseinleitungsrohr einströmen zu lassen. Die Reaktionstemperatur kann dabei im Bereich von 0 bis 50, vorzugsweise 5 bis 15°C liegen. Gegebenenfalls kann man die Phasentrennung durch Zugabe einer entsprechenden Menge Wasser schon an dieser Stelle unterstützen.

### Wäsche und Desodorierung

Durch die Behandlung mit den Sulfiermitteln reagiert die Squalanfraktion sauer. Es empfiehlt sich daher, die Wäsche, mit der die gebildeten sulfierten Paraffine entfemt werden, mit basischer Sodalösung durchzuführen, bis das Waschwasser neutral reagiert. Anschließend kann mehrfach mit Wasser nachgewaschen werden. in vielen Fällen endet das Verfahren damit, daß das Phytosqualan nach der Wäsche bis zur Gewichtskonstanz getrocknet wird. Falls gewünscht, kann jedoch noch eine zusätzliche Desodorierung eingeschoben werden, bei der man das Phytosqualan mit heißem Wasserdampf behandelt, um in Spuren vorhandene, wasserdampfflüchtige Geruchsfräger abzutrennen. Entsprechende kontinuierliche wie diskontinuierliche Verfahren sind aus dem Stand der Technik hinreichend bekannt.

Schlußendlich wird das Phytosqualan in Mengen von etwa 5 Gew.-% - bezogen auf die Einsatzstoffe - und etwa 100 Gew.-% - bezogen auf den Squalengehalt der Einsatzstoffe - erhalten. Die lodzahl der Produkte liegt unter 0,5, die Reinheit oberhalb von 99,5 %.

### Gewerbliche Anwendbarkeit

Die nach dem erfindungsgemäßen Verfahren erhältlichen Phytosqualane eignen sich als Ökörper zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/Fett-Massen, Stiftpräparate, Puder oder Salben dienen. Diese Mittel können femer als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristyimyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. Dialkylcyclohexane in Betracht.

Als **Emulgatoren** kommen beispielsweise nichtionogene Tenside aus mindestens einer der folgenden Gruppen in Frage:
- (1): Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- (2): C_{12/18}-Fettsäuremono- und -diester von Anlagerungsprodukten von 1 bis 30 Mol Ethylenoxid an Glycerin;
- (3): Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
- (4): Alkylmono- und -oligoglycoside mit 8 bis 22 Kohlenstoffatomen im Alkylrest und deren ethoxylierte Analoga;
- (5): Anlagerungsprodukte von 15 bis 60 Mol Ethylenoxid an Ricinusöl und/oder gehärtetes Ricinusöl;
- (6): Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
- (7): Anlagerungsprodukte von 2 bis 15 Mol Ethylenoxid an Ricinusöl und/oder gehärtetes Ricinusöl;
- (8): Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
- (9): Mono-, Di- und Trialkylphosphate sowie Mono-, Di- und/oder Tri-PEG-alkylphosphate und deren Salze;
- (10): Wollwachsalkohole;
- (11): Polysiloxan-Polyalkyl-Polyether-Copolymere bzw. entsprechende Derivate;
- (12): Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
- (13): Polyalkylenglycole sowie
- (14): Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte amphotytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.
Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.
Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

Typische Beispiele für **Fette** sind Glyceride, als **Wachse** kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R.Lochhead in **Cosm. Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Uchtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol,

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methytcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Famesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Als Ausgangsmaterial für die Herstellung von Phytosqualan diente ein technischer Rückstand aus der Olivenölherstellung ("skam"), der 80 Gew.-% Fettsäuren bzw. Glyceride und 20 Gew.-% unverseifbare Anteile (davon 90 Gew.-% ungesättigte Kohlenwasserstoffe, 5 Gew.-% gesättigte Kohlenwasserstoffe und 5 Gew.-% Alkohole, Terpene, Tocopherole und Stanole) enthielt.

**Beispiel 1.** In einem 3-I-Dreihalskolben mit Rührer, Rückflußkühler und Wasserabscheider wurden 1000 g des Olivenölrückstandes, enthaltend 3,6 mol freie oder gebundene Fettsäuren, 241 g (1,8 mol) Trimethylolpropan und 1 g Zinkoxalat (Frascat® 2001) vorgelegt. Zur Veresterung der freien Fettsäuren bzw. zur Umesterung der Glyceride wurde die Mischung unter Rückfluß erhitzt, wobei man die Temperatur schrittweise von 150 auf 190°C steigerte, während der Druck kontinuierlich von 750 auf 200 mbar abgesenkt wurde. Innerhalb von 8 h wurden 59 g Wasser, entsprechend 91 % der Theorie abgeschieden. Anschließend wurde der Wasserabscheider gegen einen Destillationsaufsatz ausgetauscht und die Mischung zunächst bei 290°C und 25 mbar und dann bei 250°C und 5 mbar destilliert. Während der rohe Trimethylolpropanester als Rückstand zurückblieb, wurden 102 g einer Fraktion erhalten, die im wesentlichen aus Alkoholen und Squalen bestand. Zur Überführung von Squalen in Squalan wurde die Fraktion in einen Druckreaktor gegeben, mit 0,1 Gew.-% eines Pd(C)-Katalysators (G-75, Süd-Chemie) versetzt und 1 h bei 180°C und 20 bar hydriert. Anschließend wurde der Autoklav abgekühlt, entspannt und der Katalysator abfiltriert. Das Hydrierprodukt wies danach eine Rest-lodzahl von 0,4 auf. Anschließend wurden 50 ml des Hydrierproduktes in 100 ml Hexan gelöst, auf -20°C abgekühlt und dort 5 h gelagert. Die dabei als Feststoffe ausgefallenen Anteil an noch vorhandenen Paraffinen wurden abfiltriert und das Lösemittel destillativ (40°C, 100 mbar) abgetrennt. Die gereinigte Squalanfraktion (Verseifungszahl VZ = 7,7, lodzahl IZ = 2,12, Säurezahl SZ = 3,78) wurde in einen 250-ml-Kolben überführt, dann bei 30°C mit gasförmigem Schwefeltrioxid in Form einer 0,13 Vol-%igen Lösung in Luft (molares Verhältnis Squalan : SO₃ = 1 : 0,05) behandelt und zur Abtrennung von Fettstoffen 10 min gerührt. Anschließend wurde das Reaktionsgemisch zunächst mit Sodalösung bis zum Neutralpunkt und dann mehrfach mit Wasser gewaschen und dann filtriert. Den Abschluß bildete eine Behandlung des so gereinigten Squalans (VZ = 0, IZ = 1,23, SZ = 0,07) mit Wasserdampf zur Entfernung von Spuren unerwünschter Geruchsträger sowie die Trocknung des Endproduktes bis zur Gewichtskonstanz im Trockenschrank. Es wurden 46 g Phytosqualan in einer Reinheit von 99,6 % erhalten.

**Beispiel 2.** Beispiel 1 wurde wiederholt, jedoch an Stelle des Schwefeltrioxids eine entsprechende Menge Chlorsulfonsäure verwendet. Nach der weiteren Aufarbeitung wurden ebenfalls 46 g Phytosqualan einer Reinheit von 99,6 Gew.-% erhalten.

**Beispiel 3.** Beispiel 1 wurde wiederholt, jedoch anstelle von Trimethylolpropan eine entsprechende molare Menge Pentaerythrit eingesetzt. Die Veresterung/Umesterung wurde über einen Zeitraum von 10 h durchgeführt, wobei 60 g Wasser, entsprechend einem Umsatz von 92 % der Theorie abgeschieden wurden. Nach Aufarbeitung der Squalen/Squalanphase wurden 41 g Phytosqualan in einer Reinheit von 99,6 % erhalten.

**Vergleichsbeispiel V1.** Beispiel 1 wurde wiederholt, die Deparaffinierung jedoch anstelle von Schwefeltrioxid mit Schwefelsäure (54 Gew.-%ig) durchgeführt. Es wurden 38 g Phytosqualan in einer Reinheit von 97,4 % erhalten.

**Vergleichsbeispiel V2.** Beispiel 1 wurde wiederholt, die fraktionierte Destillation jedoch vor der Hydrierung durchgeführt. Es wurden 40 g Phytosqualan in einer Reinheit von 97,1 % erhalten.

## Patentansprüche

1. Verfahren zur Herstellung von Phytosqualan, **dadurch gekennzeichnet, daß** man
(a) einen phytosqualenhaltigen Rückstand der Pflanzenölherstellung mit einem Polyol zur Derivatisierung des Fettsäureanteils verestert,
(b) die resultierende Reaktionsmischung in eine Polyolesterfraktion (Rückstand) und ein phytosqualenhaltiges Destillat trennt,
(c) das Destillat unter Erzeugung einer squalanreichen Fraktion hydriert,
(d) das resultierende flüssige Hydrierprodukt einer fraktionierten Kristallisation unterwirft,
(e) die von Paraffinen befreite flüssige Phase mit Schwefeltrioxid, Chlorsulfonsäure oder Amidosulfonsäure behandelt, und
(f) aus dem Rückstand das reine Phytosqualan durch Wäsche und Desodorierung gewinnt.

2. Verfahren nach Anspruch 1, **dadurch gekennzeichnet, daß** man Rückstände aus der Olivenölherstellung einsetzt.

3. Verfahren nach Anspruch 1 oder 2, **dadurch gekennzeichnet, daß** man Polyole einsetzt, ausgewählt aus der Gruppe, die gebildet wird von technischen Oligoglyceringemischen, Methylolverbindungen, Niedrigalkylglucosiden, Zuckeralkoholen, Zuckern und Aminozuckern.

4. Verfahren nach mindestens einem der Ansprüche 1 bis 3, **dadurch gekennzeichnet, daß** man die Rückstände und die Polyole - bezogen auf die zur Verfügung stehenden Acylreste und die Hydroxylgruppen - im molaren Verhältnis 1 : 2 bis 1 : 3. einsetzt.

5. Verfahren nach mindestens einem der Ansprüche 1 bis 4, **dadurch gekennzeichnet, daß** man als Veresterungs- bzw. Umesterungskatalysatoren Zinkseifen in Mengen von 0,1 bis 5 Gew.-% einsetzt.

6. Verfahren nach mindestens einem der Ansprüche 1 bis 5, **dadurch gekennzeichnet, daß** man die Veresterung bzw. Umesterung bei Temperaturen von 130 bis 220°C sowie verminderten Drücken im Bereich von 0,9 bis 0,05 bar durchführt.

7. Verfahren nach mindestens einem der Ansprüche 1 bis 6, **dadurch gekennzeichnet, daß** man die Abtrennung der leichtsiedenden squalenhaltigen Fraktion von den schwersiedenden Polyolestern bei Temperaturen im Bereich von 250 bis 290°C und Drücken von 5 bis 25 mbar durchführt.

8. Verfahren nach mindestens einem der Ansprüche 1 bis 7, **dadurch gekennzeichnet, daß** man die Hydrierung in Gegenwart von Nickel-, Palladium- oder Platinkatalysatoren bei Temperaturen in Bereich von 150 bis 200 °C und Drücken von 1 bis 25 bar durchgeführt.

9. Verfahren nach mindestens einem der Ansprüche 1 bis 8, **dadurch gekennzeichnet, daß** man die fraktionierte Kristallisation, gegebenenfalls in einem inerten Lösemittel, bei -15 bis -25°C durchführt.

10. Verfahren nach mindestens einem der Ansprüche 1 bis 9, **dadurch gekennzeichnet, daß** man die Squalanfraktion mit 0,1 bis 10 Gew.-% des Sulfiermittels behandelt.

## Claims

1. A process for the production of phytosqualane, **characterized in that**
(a) a phytosqualene-containing residue from the production of vegetable oils is esterified with a polyol to derivatize the fatty acid component,
(b) the resulting reaction mixture is separated into a polyol ester fraction (residue) and a phytosqualene-containing distillate,
(c) the distillate is hydrogenated to produce a fraction rich in squalane,
(d) the resulting liquid hydrogenation product is subjected to fractional crystallization,
(e) the liquid phase freed from paraffins is treated with sulfur trioxide, chlorosulfonic acid or amidosulfonic acid and
(f) the pure phytosqualane is obtained from the residue by washing and deodorization.

2. A process as claimed in claim 1, **characterized in that** residues from the production of olive oil are used.

3. A process as claimed in claim 1 or 2, **characterized in that** polyols selected from the group consisting of technical oligoglycerol mixtures, methylol compounds, lower alkyl glucosides, sugar alcohols, sugars and amino sugars are used.

4. A process as claimed in at least one of claims 1 to 3, **characterized in that** the residues and the polyols, based on the acyl groups available and the hydroxyl groups, are used in a molar ratio of 1:2 to 1:3.

5. A process as claimed in at least one of claims 1 to 4, **characterized in that** zinc soaps in quantities of 0.1 to 5% by weight are used as esterification or transesterification catalysts.

6. A process as claimed in at least one of claims 1 to 5, **characterized in that** the esterification or transesterification is carried out at temperatures of 130 to 220°C and under reduced pressures of 0.9 to 0.05 bar.

7. A process as claimed in at least one of claims 1 to 6, **characterized in that** the low-boiling squalene-containing fraction is separated from the high-boiling polyol esters at temperatures of 250 to 290°C and under pressures of 5 to 25 mbar.

8. A process as claimed in at least one of claims 1 to 7, **characterized in that** the hydrogenation is carried out in the presence of nickel, palladium or platinum catalysts at temperatures of 150 to 200°C and under pressures of 1 to 25 bar.

9. A process as claimed in at least one of claims 1 to 8, **characterized in that** the fractional crystallization is carried out at -15°C to -25°C, optionally in an inert solvent.

10. A process as claimed in at least one of claims 1 to 9, **characterized in that** the squalane fraction is treated with 1 to 10% by weight of the sulfonating agent.

## Revendications

1. Procédé de préparation de phytosqualane,
**caractérisé en ce qu'**
a) on estérifie un résidu contenant du phytosqualène de la production d'huile végétale avec un polyol en vue de produire un dérivé de la fraction acide gras,
b) on sépare le mélange réactionnel résultant en une fraction ester de polyol (résidu) et un distillat contenant du phytosqualène,
c) on hydrogène le distillat en produisant une fraction riche en squalane,
d) on soumet le produit d'hydrogénation liquide résultant à une cristallisation fractionnée,
e) on traite les phases liquides débarrassées des paraffines avec de l'anhydride sulfurique, de l'acide chlorosufonique ou de l'acide amidosulfonique. et
f) on récupère du résidu le phytosqualane pur par lavage et désodorisation.

2. Procédé selon la revendication 1,
**caractérisé en ce qu'**
on met en oeuvre des résidus provenant de la production d'huile d'olive.

3. Procédé selon la revendication 1 ou la revendication 2,
**caractérisé en ce qu'**
on met en oeuvre des polyols choisis dans le groupe formé des mélanges industriels d'oligoglycérols, de composés méthyloliques, glucosides, d'alkyl inférieur, d'alcools de sucre, de sucres et d'amino-sucres.

4. Procédé selon au moins une des revendications 1 à 3,
**caractérisé en ce qu'**
on met en oeuvre les résidus et les polyols - rapportés aux restes acyle mis à disposition et aux groupes hydroxyle - dans un rapport molaire 1 : 2 à 1 : 3.

5. Procédé selon au moins une des revendications 1 à 4,
**caractérisé en ce qu'**
on met en oeuvre comme catalyseurs d'estérification ou de transesterification des savons de zinc en quantités de 0,1 à 5% en poids.

6. Procédé selon au moins une des revendications 1 à 5,
**caractérisé en ce qu'**
on effectue l'estérification ou la transesterification à des températures de 130 à 220°C ainsi qu'à des pressions réduites dans la zone de 0,9 à 0,05 bar.

7. Procédé selon au moins une des revendications 1 à 6,
**caractérisé en ce que**
la séparation de la fraction contenant du squalène à bas point d'ébullition, des esters de polyols qui bouent difficilement est effectué à des températures dans la zone de 250 à 290°C et à des pressions de 5 à 25 mbars.

8. Procédé selon au moins une des revendications 1 à 7,
**caractérisé en ce qu'**
on effectue l'hydrogénation en présence de catalyseurs au nickel, au palladium ou au platine à des températures dans la zone de 150 à 200°C et à des pressions de 1 à 25 bars.

9. Procédé selon au moins une des revendications 1 à 8,
**caractérisé en ce qu'**
on effectue la cristallisation fractionnée, le cas échéant dans un solvant inerte, à -15 à -25°C.

10. Procédé selon au moins une des revendications 1 à 9
**caractérisé en ce qu'**
on traite la fraction squalane avec de 0,1 à 10% en poids de l'agent de sulfatation.
